(19) Europäisches Patentamt
European Patent Office
Office européen des brevets

(11) Veröffentlichungsnummer: **0 092 095 A2**

# (12) EUROPÄISCHE PATENTANMELDUNG

(21) Anmeldenummer: **83103306.3**

(22) Anmeldetag: **05.04.83**

(51) Int. Cl.³: **C 07 D 233/90**, A 01 N 43/50

(30) Priorität: **17.04.82 DE 3214227**

(43) Veröffentlichungstag der Anmeldung: **26.10.83 Patentblatt 83/43**

(84) Benannte Vertragsstaaten: **AT BE CH DE FR GB IT LI NL SE**

(71) Anmelder: **BASF Aktiengesellschaft, Carl-Bosch-Strasse 38, D-6700 Ludwigshafen (DE)**

(72) Erfinder: **Rentzea, Costin, Dr., Neuenheimer Landstrasse 72, D-6900 Heidelberg (DE)**
Erfinder: **Spiegler, Wolfgang, Dr., Amsterdamer Strasse 16, D-6700 Ludwigshafen (DE)**
Erfinder: **Pommer, Ernst-Heinrich, Dr., Berliner Platz 7, D-6703 Limburgerhof (DE)**
Erfinder: **Ammermann, Eberhard, Dr., Sachsenstrasse 3, D-6700 Ludwigshafen (DE)**

(54) **1-(Trihalogenmethyl-sulfenyl)-4-methylimidazol-5-carboxamide und diese enthaltende Fungizide.**

(57) 1-(Trihalogenmethyl-sulfenyl)-4-methylimidazol-5-carboxamide der allgemeinen Formel

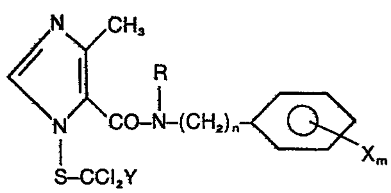

(I)

in der

R ein Wasserstoffatom oder einen Methyl-, Ethyl- oder Propylrest bedeutet,

X ein Wasserstoff- oder Halogenatom, einen gegebenenfalls durch Fluor, Chlor oder Brom substituierten Alkyl- oder Alkoxyrest mit 1 bis 5 C-Atomen, einen Alkenylrest mit 2 bis 4 C-Atomen, oder einen Cyan-, Nitro-, Phenyl- oder Phenoxyrest bedeutet, wobei der Phenyl- oder der Phenoxyrest durch Fluor, Chlor oder Brom oder Alkyl mit 1 bis 4 C-Atomen substituiert sein kann,

m gleich 1 oder 2 ist und

n gleich 0, 1 oder 2 ist und

Y ein Fluor- oder Chloratom bedeutet und Mittel zur Bekämpfung von Pilzen.

EP 0 092 095 A2

1-(Trihalogenmethyl-sulfenyl)-4-methylimidazol-5-carooxamide und diese enthaltende Fungizide

Die vorliegende Erfindung betrifft neue wertvolle 1-(Trihalogenmethyl-sulfenyl)-4-methylimidazol-5-carboxamide mit fungizider Wirkung sowie deren Verwendung zur Bekämpfung phytopathogener Pilze und Fungizide, die diese Verbindungen enthalten.

Es ist bekannt, N-Trichlormethylthio-tetrahydrophthalimid zur Bekämpfung von gewissen phytopathogenen Pilzen zu verwenden (Chemical Week 1972, Juni 21, Seite 63). Seine Wirkung ist jedoch gegen andere Phycomyceten, z.B. Phytophthora infestans bei Tomaten oder Kartoffeln nicht befriedigend. Zum Materialschutz oder zum Schutz von Holz gegen holzzerstörende Pilze genügt seine Wirkung nicht.

Es wurde nun gefunden, daß neue 1-(Trihalogenmethyl--sulfenyl)-4-methylimidazol-5-carboxamide der allgemeinen Formel

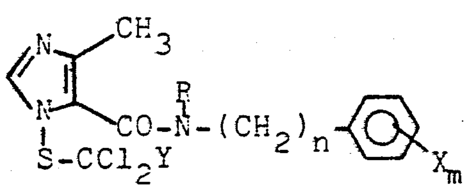

(I)

in der

R ein Wasserstoffatom oder einen Methyl-, Ethyl- oder Propylrest bedeutet,

X ein Wasserstoff- oder Halogenatom, einen gegebenenfalls durch Fluor, Chlor oder Brom substituierten Alkyl- oder Alkoxrest mit 1 bis 5 C-Atomen, einen Alkenyl-Sws/ro

rest mit 2 bis 4 C-Atomen, oder einen Cyan-, Nitro-, Phenyl- oder Phenoxyrest bedeutet, wobei der Phenyl- oder der Phenoxyrest durch Fluor, Chlor oder Brom oder Alkyl mit 1 bis 4 C-Atomen substituiert sein kann,
m gleich 1 oder 2 ist,
n gleich 0, 1 oder 2 ist und
Y ein Fluor- oder Chloratom bedeutet, eine starke fungizide Wirkung haben.

Die neuen Stoffe haben ein breites Wirkungsspektrum und können angewandt werden vor allem gegen Phycomyceten und Fungi imperfecti aber auch gegen Ascomyceten. Die neuen Verbindungen sind beispielsweise geeignet zur Anwendung im Pflanzenschutz zur Bekämpfung phytopathogener Pilze. Dabei schädigen sie Kulturpflanzen in den zur Bekämpfung von Pilzen notwendigen Konzentrationen nicht. Die neuen 1-(Trihalogenmethyl-sulfenyl)-4-methylimidazol-5-carboxamide eignen sich ferner hervorragend zum Materialschutz und zum Schutz von Holz.

Weiterhin wurde gefunden, daß man die Verbindungen der allgemeinen Formel (I) erhält, wenn man ein 4-Methylimidazol-5-carboxamid der Formel (II)

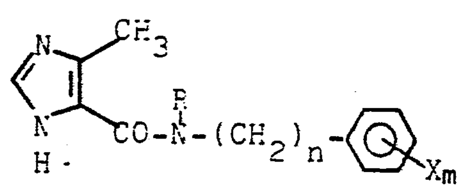

(II)

mit Sulfenylchloriden der Formel $YCCl_2-S-Cl$ (III), wobei R, X, Y, m und n die oben angegebenen Bedeutungen haben, gegebenenfalls in Gegenwart eines Säurebindemittels und gegebenenfalls in Gegenwart eines Lösungs- bzw. Verdünnungsmittels umsetzt. Die Umsetzungen werden zweckmäßig in

gegenüber den Reaktionsteilnehmern inerten Lösungs- oder Verdünnungsmitteln, z.B. Wasser, Toluol, Xylol, Diethylether, Tetrahydrofuran, Dioxan, Dimethoxyethan, Aceton, Methylethylketon, Essigsäureester, Methylenchlorid, Chloroform, Dichlorethan oder Chlorbenzol ein- oder zweiphasig durchgeführt.

Als säurebindende Mittel kommen z.B. anorganische Basen wie Hydroxide oder Carbonate von Alkali- und Erdalkalimetallen (z.B. NaOH, $NaHCO_3$, $KHCO_3$, $K_2CO_3$, $CaCO_3$, $BaCO_3$) und vor allem tertiäre Amine, wie Triethylamin, N,N-Dimethyl-cyclohexylamin, N,N-Dimethylanilin oder Pyridin in Frage.

Die Umsetzungen laufen bei einer Temperatur schon oberhalb von -30 und im allgemeinen unter +100°C ab, vorzugsweise arbeitet man bei einer möglichst niedrigen Temperatur, etwa zwischen -10 und +25°C und bei atmosphärischem Druck.

Solche Verbindungen der allgemeinen Formel (I), in denen Y Fluor bedeutet, kann man auch aus einer Verbindung der Formel Ia

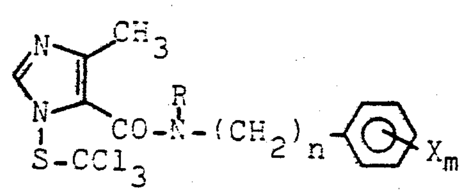

(Ia)

in der R, X, m und n die oben angegebenen Bedeutungen haben, mit wasserfreier Fluorwasserstoffsäure erhalten, wobei in der Trichlormethylthio-Seitenkette ein Chloratom gegen Fluor

ausgetauscht werden kann. Diese Umsetzung kann in einem Überschuß von Fluorwasserstoffsäure als Verdünnungsmittel bei einer Temperatur beispielsweise zwischen -50 und +80°C, vorzugsweise zwischen -10 und +25°C unter atmosphärischem oder erhöhtem Druck durchgeführt werden.

X bedeutet in diesem Falle vorzugsweise Wasserstoff, Fluor, Chlor, Brom, Methyl, Ethyl, Isopropyl, tert.-Butyl, Trifluormethyl, Nitro, Methoxy, Ethoxy, Tetrafluorethoxy, Phenoxy und Phenyl.

R und n haben die oben angegebene Bedeutung.

Die als Ausgangsstoffe zu verwendenden 4-Aryl-1,2,4-triazolidin-5-one sind aus J. Org. Chem., 29, 3707 (1964) bekannt oder können nach dem dort beschriebenen Verfahren erhalten werden.

Ausgangsstoffe (II), die sich für die Herstellung entsprechender Verbindungen (I) eignen, sind in der Tabelle 1 im einzelnen beschrieben; soweit ihre Schmelzpunkte (Fp) angegeben sind, wurden sie auch jeweils hergestellt.

Die weiterhin für die Herstellung der neuen Stoffe erforderlichen 1-Trichlormethyl-sulfenyl-4-methylimidazol-5-carboxamide sind durch die Formel (Ia) allgemein definiert. Sie können ebenfalls nach bekannten Verfahren hergestellt werden. Angaben dazu finden sich bei den Herstellungsbeispielen.

Die schließlich für die Herstellung der erfindungsgemäßen Stoffe erforderlichen Trihalogenmethyl-sulfenyl-chloride (III) sind allgemein bekannt.

Tabelle 1

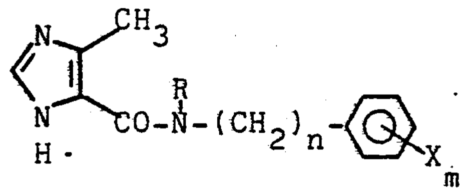

(II)

| $X_m$ | R | n | Fp [°C] |
|---|---|---|---|
| H | H | 0 | 265-267 |
| H | $-CH_3$ | 0 | 199-201 |
| H | $-C_2H_5$ | 0 | 130-132 |
| H | H | 1 | 135-137 |
| H | H | 2 | 123-124 |
| 4-F | H | 0 | 194-195 |
| 4-F | H | 1 | 168-170 |
| 4-Cl | H | 0 | 213-215 |
| 4-Cl | H | 1 | 196-198 |
| 4-Cl | $CH_3$ | 1 | 185-187 |
| 3-Cl | H | 0 | 214-216 |
| 4-Br | H | 0 | 231-235 |
| $4-CH_3$ | H | 0 | 208-210 |
| $4-CH_3$ | H | 1 | 190-192 |
| $4-CH_3$ | H | 2 | 170-172 |
| $4-CH_3$ | $n-C_3H_7$ | 0 | |
| $4-CH_3$ | $C_2H_5$ | 1 | |
| $4-CH_3O-$ | H | 0 | 235-236 |
| $4-CH_3O-$ | H | 1 | 177-179 |
| $4-CH_3O-$ | H | 2 | |
| $4-CH_3O-$ | $CH_3$ | 0 | |
| $4-NO_2$ | H | 0 | |
| $4-NO_2$ | H | 1 | |
| $4-C_2H_5-O-$ | H | 0 | 246-248 |

| $X_m$ | R | n | Fp [°C] |
|---|---|---|---|
| $3-C_2H_5-O-$ | H | 0 | 173-175 |
| $2-C_2H_5-O-$ | H | 0 | 160-162 |
| $4-C_2H_5-O-$ | H | 1 | |
| $3-CHF_2-CF_2-O$ | H | 0 | 167-169 |
| $4-CF_3$ | H | 0 | |
| $3-CF_3$ | H | 0 | |
| $3,5-(CF_3)_2$ | H | 0 | |
| $3-C_3H_7-n$ | H | 0 | 150-151 |
| $4-C_3H_7-n$ | H | 0 | |
| $4-C_3H_7-i$ | H | 0 | |
| $4-(tert.-C_4H_9)$ | H | 0 | |
| $2,4-Cl_2$ | H | 1 | 170-173 |
| $3,5-(CH_3O)_2$ | H | 0 | 173-175 |
| $3-Cl, 4-CH_3O$ | H | 0 | 222-224 |
| $3,4-Cl_2$ | H | 0 | |
| $3,5-Cl_2$ | H | 0 | |
| $3,5-Cl_2, 4-CH_3O$ | H | 0 | |
| $3-Cl, 4-F$ | H | 0 | |
| $4-C_6H_5$ | H | 0 | |
| $4-C_6H_5-O$ | H | 0 | |
| $2,5-(C_2H_5O)_2$ | H | 0 | 139-141 |
| $3-C_2H_5$ | H | 0 | Harz |

Herstellungsbeispiele

Beispiel 1

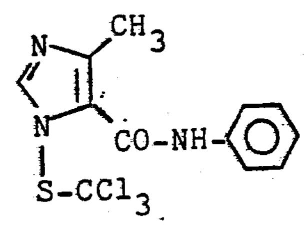

Zu einer Suspension von 20,1 g (0,1 Mol) 4-Methylimidazol-5-carboxanilid in 150 ml trockenem Essigester wurden bei etwa 10 bis 15°C nacheinander 19 g (0,102 Mol) Perchlormethylmercaptan und 10 g (0,099 Mol) Triethylamin unter gutem Rühren nach und nach zugesetzt. Nach zweistündigem Rühren bei Raumtemperatur (20°C) konnte das ausgefallene Triethylaminhydrochlorid abgesaugt und mit 40 ml Essigester gewaschen werden. Das Filtrat wurde zweimal mit je 100 ml Wasser gewaschen (ausgeschüttelt), über $Na_2SO_4$ getrocknet und eingeengt. Der Rückstand kristallisierte bei 0°C nach Zugabe von 20 ml Ether.
Man erhielt 18,7 g (53,3 % d. Th.) 1-Trichlormethyl-sulfenyl-4-methyl-5-carboxanilid als weiße Kristalle vom Schmelzpunkt 120 bis 122°C. (Nr. 1)

Beispiel 2

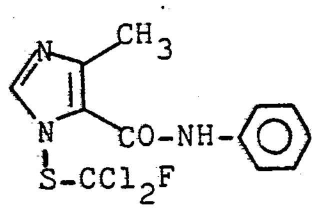

Nach den Angaben in Beispiel 1 wurden 20,1 g (0,1 Mol) 4-Methyl-imidazol-5-carboxanilid mit 17 g (0,1 Mol) Fluordichlormethyl-sulfenylchlorid umgesetzt. Man erhielt 9,4 g (28 % d.Th.) 1-Fluordichlormethyl-sulfenyl-4-methyl-imidazol-5-carboxanilid als weiße Kristalle vom Schmelzpunkt 91 bis 92°C. (Nr. 2)

Entsprechend wurden diejenigen in der Tabelle 2 angegebenen Verbindungen der Formel I hergestellt, deren Schmelzpunkte (Fp) angegeben sind ihre Struktur wurde durch Ultrarot- und Kernresonanz-Spektroskopie sowie durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden; es kann erwartet werden, daß sie aufgrund ihrer ähnlichen Konstitution ähnliche Wirkungen wie die näher untersuchten Verbindungen haben.

Tabelle 2

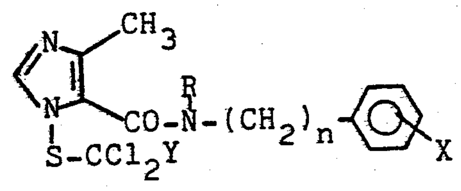

(I)

| Nr.- | $X_m$ | R | n | Y | Fp [°C] |
|---|---|---|---|---|---|
| 3 | H | $CH_3$ | 0 | Cl | 115-117 |
| 4 | H | $CH_3$ | 0 | F | |
| 5 | H | $C_2H_5$ | 0 | Cl | |
| 6 | H | H | 1 | Cl | 126-128 |
| 7 | H | H | 2 | Cl | 106-107 |
| 8 | H | H | 2 | F | Harz |
| 9 | 4-F | H | 0 | Cl | |
| 10 | 4-F | H | 1 | Cl | 120-122 |
| 11 | 4-Cl | H | 0 | Cl | 175-177 |
| 12 | 4-Cl | H | 1 | Cl | 140-141 |
| 13 | 4-Cl | H | 1 | F | |
| 14 | 4-Cl | $CH_3$ | 1 | Cl | |
| 15 | 4-Br | H | 0 | Cl | 195-197 |
| 16 | 4-Br | H | 0 | F | |
| 17 | $4-CH_3$ | H | 0 | Cl | 172-174 |
| 18 | $4-CH_3$ | H | 0 | F | |
| 19 | $4-CH_3$ | $n-C_3H_7$ | 0 | Cl | |
| 20 | $4-CH_3$ | H | 1 | Cl | 154-156 |
| 21 | $4-CH_3$ | $C_2H_5$ | 1 | Cl | |
| 22 | $4-CH_3O-$ | H | 0 | Cl | 121-123 |
| 23 | $4-CH_3O-$ | H | 1 | Cl | 133-135 |
| 24 | $4-NO_2$ | H | 0 | Cl | |
| 25 | $4-NO_2$ | H | 1 | Cl | |
| 26 | $4-NO_2$ | H | 0 | F | |

| Nr.- | $X_m$ | R | n | Y | Fp [°C] |
|---|---|---|---|---|---|
| 27 | $4-C_2H_5-O$ | H | 0 | Cl | 130-133 |
| 28 | $4-C_2H_5-O$ | H | 0 | F | |
| 29 | $3-C_2H_5-O$ | H | 0 | Cl | 115-117 |
| 30 | $3-CHF_2-CF_2-O$ | H | 0 | Cl | 100-102 |
| 31 | $3-CHF_2-CF_2-O$ | H | 0 | F | |
| 32 | $2-C_2H_5-O$ | H | 0 | Cl | 160-163 |
| 33 | $2-C_2H_5-O$ | H | 0 | F | |
| 34 | $3-C_2H_5$ | H | 0 | Cl | 100-102 |
| 35 | $3-C_3H_7-n$ | H | 0 | Cl | 115-117 |
| 36 | $4-C_3H_7-n$ | H | 0 | Cl | |
| 37 | $4-C_3H_7-n$ | H | 0 | F | |
| 38 | $4-C_3H_7-i$ | H | 0 | Cl | |
| 39 | 4-(tert.-$C_4H_9$) | H | 0 | Cl | |
| 40 | $3,5-(CH_3O)_2$ | H | 0 | Cl | 139-140 |
| 41 | $2,4-(CH_3O)_2$ | H | 0 | Cl | |
| 42 | $4-CH_3$, $3-CH_3O$ | H | 0 | Cl | 153-154 |
| 43 | 3-Cl, $4-CH_3O$ | H | 0 | Cl | 157-160 |
| 44 | $3,4-Cl_2$ | H | 0 | Cl | |
| 45 | $3,5-Cl_2$ | H | 0 | Cl | |
| 46 | $2,4-Cl_2$ | H | 0 | Cl | |
| 47 | $2,4-Cl_2$ | H | 1 | Cl | 113-115 |
| 48 | $2,4-Cl_2$ | H | 1 | F | |
| 49 | $4-C_6H_5$ | H | 0 | Cl | |
| 50 | $4-C_6H_5-O$ | H | 0 | Cl | |

Die neuen Verbindungen zeichnen sich durch eine hervorragende Wirksamkeit gegen ein breites Spektrum von pflanzenpathogenen Pilzen aus. Sie können als Blatt- und Bodenfungizide eingesetzt werden.

Die neuen Verbindungen sind z.B. geeignet zur Bekämpfung von Pflanzenkrankheiten, wie

Phytophthora infestans an Tomaten und Kartoffeln,
Phytophthora parasitica an Erdbeeren,
Phytophthora cactorum an Äpfeln,
Pseudoperonospora cubensis an Gurken,
Pseudoperonospora humuli an Hopfen,
Peronospora destructor an Zwiebeln,
Peronospora sparsa an Rosen,
Peronospora tabacina an Tabak,
Plasmopara viticola an Reben,
Plasmopara halstedii an Sonnenblumen,
Pythium ultimum an Erbsenkeimlingen,
Botrytis cinerea an Reben, Erdbeeren und Paprika,
Septoria nodorum an Getreide,
Venturia inaequalis (Schorf) an Apfelbäumen.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate, Die Anwendungsformen richten sich ganz nach den Verwendungszwecken; sie sollten in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere

organische Lösungsmittel als Hilfslösungsmittel verwendet werden können. Als Hilfsstoffe kommen dafür im wesentlichen in Frage: Lösungsmittel wie Aromaten (z.B. Xylol, Benzol), chlorierte Aromaten (z.B. Chlorbenzole), Paraffine (z.B. Erdölfraktionen), Alkohole (z.B. Methanol, Butanol), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,1 und 3 kg Wirkstoff oder mehr je ha. Die neuen Verbindungen können auch in Materialschutz eingesetzt werden. Bei der Anwendung der Wirkstoffe im Materialschutz betragen die Aufwandmengen 0,05 bis 5 % (Gew.%) Wirkstoff, bezogen auf das Gesamtgewicht des zu konservierenden Materials. Die neuen Wirkstoffe können auch als fungizid wirksame Bestandteile öliger Holzschutzmittel zum Schutz von Holz gegen holzverfärbende Pilze eingesetzt werden. Die Anwendung erfolgt in der Weise, daß man das Holz mit diesen Mitteln behandelt, beispielsweise tränkt oder anstreicht.

Die Mittel bzw. die daraus hergestellten gebrauchsfertigen Zubereitungen wie Lösungen, Emulsionen, Suspensionen, Pulver, Stäube, Pasten oder Granulate werden in bekannter

Weise angewendet, beispielsweise durch Versprühen, Vernebeln, Verstäuben, Verstreuen, Beizen oder Gießen.

Angaben zur Formulierung (Herstellung von Zubereitungen) der erfindungsgemäßen Stoffe können beispielsweise der DE-OS 30 25 879 bzw. der EP-OS 00 44.407 entnommen werden.

Fungizide, die mit den erfindungsgemäßen Verbindungen kombiniert werden können, sind beispielsweise:

Elementarer Schwefel,
Dithiocarbamate und deren Derivate, wie
Ferridimethyldithiocarbamat,
Zinkdimethyldithiocarbamat,
Mangan-Zink-ethylendiamin-bis-dithiocarbamat und

Zinkethylenbisdithiocarbamat,
Tetramethylthiuramdisulfide,
Ammoniak-Komplex von Zink-(N,N-ethylen-bis-dithiocarbamat) und N,N'-Polyethylen-bis-(thiocarbamoyl)-disulfid,
Zink-(N,N'-propylen-bis-dithiocarbamat),
Ammoniak-Komplex von Zink-(N,N'-propylen-bis-dithiocarbamat) und
N,N'-Polypropylen-bis-(thiocarbamoyl)-disulfid;

Nitroderivate, wie
Dinitro-(1-methylheptyl)-phenylcrotonat,
2-sec.-Butyl-4,6-dinitrophenyl-3,3-dimethylacrylat,
2-sec.-Butyl-4,6-dinitrophenyl-isopropylcarbonat;

heterocyclische Substanzen, wie
N-(1,1,2,2-Tetrachlorethylthio=-tetrahydrophthalimid,
N-Trichlormethylthio-tetrahydrophthalimid,
2-Heptadecyl-2-imidazolin-acetat

2,4-Dichlor-6-(o-chloranilino)-s-triazin
O,O-Diethyl-phthalimidophosphothioat
5-Amino-1-(bis-(dimethylamino)-phosphinyl)-3-phenyl-1,2,4-triazol)
2,3-Dicyano-1,4-Dithioaanthrachinon
2-Thio-1,3-dithio-(4,5-b)-chinoxalin
1-(Butylcarbamoyl)-2-benzimidazol-carbaminsäuremethylester
4-(2-Chlorphenylhydrazono)-3-methyl-5-isoxazolon
Pyridin-2-thio-1-oxid
8-Hydroxychinolin bzw. dessen Kupfersalze
2,3-Dihydro-5-carboxanilido-6-methyl-1,4-oxathiin-4,4-dioxid
2,3-Dihydro-5-carboxanilido-6-methyl-1,4-oxathiin
2-(Furyl-(2))-benzimidazol
Piperazin-1,4-diylbis-(1-(2,2,2-trichlor-ethyl)-formamid
2-(Thiazolyl)-(4)-benzimidazol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol
sowie verschiedene Fungizide, wie
Dodecylguanidinacetat
3-(3-(3,5-Dimethyl-2-oxycyclohexyl)-2-hydroxyethyl)-glutarimid
Hexachlorbenzol
N-Dichlorfluormethylthio-N',N'-dimethyl-N-phenyl-schwefelsäurediamid
2,5-Dimethyl-furan-3-carbonsäureanilid
2-Methyl-benzoesäure-anilid
2-Jod-benzoesäure-anilid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
1-(1',2',4'-Triazolyl-1')-[1-(4'-chlorphenoxy)]-3,3-dimethylbutan-2-on,

1-(1',2',4'-Triazolyl-1')-[1-(4'-chlorphenoxy)]-3,3-dimethylbutan-2-ol,

DL-Methyl-N-(2,6-dimethyl-phenyl)-N-furoyl(2)-alaninat,
DL-N-(2,6-Dimethyl-phenyl)-N-(2'-methoxyacetyl)-alanin-methylester,
5-Nitro-isophthalsäure-di-isopropylester,
N-(2,6-Dimethylphenyl)-N-chloracetyl-D,L-2-aminobutyrolacton,
N-(n-Propyl)-N-(2,4,6-trichlorphenoxyethyl)-N'-imidazolyl-harnstoff,
N-Cyclohexyl-N-methoxy-2,5-dimethyl-furan-3-carbonsäureamid,
2,4,5-Trimethyl-furan-3-carbonsäureanilid,
5-Methyl-5-vinyl-3-(3,5-dichlorphenyl)-2,4-dioxo-1,3-oxazolidin,
5-Methoxymethyl-5-methyl-3-(3,5-dichlorphenyl)-2,4-dioxo-1,3-oxazolidin,
N-[3-(p-tert.-Butylphenyl)-2-methyl-propyl]-cis-2,6-dimethylmorpholin,
N-Formyl-N-morpholin-2,2,2-trichlorethylacetal
1-[2-(2,4-Dichlorphenyl)-4-ethyl-1,3-dioxolan-2-yl-methyl]-1H-1,2,4-triazol,
1-[2-(2,4-Dichlorphenyl)-4-n-propyl-1,3-dioxolan-2-yl-methyl]-1H-1,2,4-triazol.

Für die folgenden Versuche wurde der folgende bekannte Wirkstoff als Vergleichsmittel verwendet:

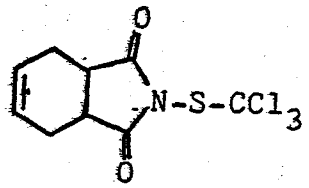 Vergleichsmittel A

Versuch 1

Wirksamkeit gegen Botrytis cinerea an Paprika

Paprikasämlinge der Sorte "Neusiedler Ideal Elite" werden, nachdem sich 4 bis 5 Blätter gut entwickelt haben, mit wäßrigen Suspensionen, die 80 % Wirkstoff und 20 % Emulgiermittel in der Trockensubstanz enthalten, tropfnaß gespritzt.

Nach dem Antrocknen des Spritzbelages werden die behandelten und einige unbehandelte Kontrollpflanzen mit einer Konidienaufschwemmung des Pilzes Botrytis cinerea besprüht und bei 22-24°C in eine Kammer mit hoher Luftfeuchtigkeit gestellt. Nach 5 Tagen hat sich die Krankheit auf den Kontrollpflanzen so stark entwickelt, daß die entstandenen Blattnekrosen den überwiegenden Teil der Blätter bedecken.

Bewertung: 0 = kein Pilzbefall, abgestuft bis
5 = Totalbefall

| Wirkstoff nach Beispiel | Befall der Blätter nach Applikation von 0,05%iger Wirkstoffbrühe |
|---|---|
| 1 | 0 |
| 6 | 0 |
| 10 | 0 |
| 12 | 0 |
| 15 | 1 |
| 17 | 1 |
| 22 | 0 |
| 23 | 0 |
| 27 | 0 |
| 29 | 1 |
| 34 | 1 |
| 40 | 0 |
| 42 | 0 |
| 43 | 0 |
| 47 | 0 |
| Vergleichsmittel | 3 |
| unbehandelte Kontrollpflanzen | 5 |

Beispiel 2

Wirksamkeit gegen Phytophthora infestans an Tomaten

Blätter von Topfpflanzen der Sorte "Große Fleischtomate" werden mit wäßriger Spritzbrühe besprüht, die 80 % Wirkstoff und 20 % Emulgiermittel in der Trockensubstanz enthält. Nach dem Antrocknen des Spritzbelages bzw. Auftrocknen von Gießwasserresten der Kontrollpflanzen werden die Blätter mit einer Zoosporenaufschwemmung des Pilzes Phytophthora infestans infiziert. Die Pflanzen werden dann in einer wasserdampfgesättigten Kammer bei Temperaturen

zwischen 16 und 18 °C aufgestellt. Nach 5 Tagen hat sich die Krankheit auf den Kontrollpflanzen so stark entwickelt, daß die fungizide Wirksamkeit der Substanzen beurteilt werden kann.

Bewertung: 0 = kein Pilzbefall, abgestuft bis
5 = Totalbefall

| Wirkstoff nach Beispiel | Befall der Blätter nach Applikation von 0,025%iger Wirkstoffbrühe |
|---|---|
| 1 | 2 |
| 6 | 0 |
| 7 | 0 |
| 20 | 2 |
| Vergleichsmittel | 3-4 |
| Unbehandelte Kontrollpflanzen | 4-5 |

Patentansprüche

1. 1-(Trihalogenmethyl-sulfenyl)-4-methylimidazol-5-carboxamid der allgemeinen Formel

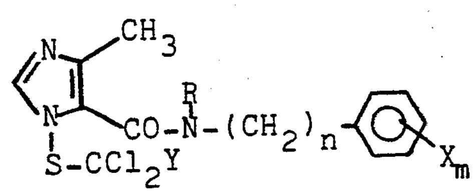

(I)

in der
R ein Wasserstoffatom oder einen Methyl-, Ethyl- oder Propylrest bedeutet,
X ein Wasserstoff- oder Halogenatom, einen gegebenenfalls durch Fluor, Chlor oder Brom substituierten Alkyl- oder Alkoxyrest mit 1 bis 5 C-Atomen, einen Alkenylrest mit 2 bis 4 C-Atomen, oder einen Cyan-, Nitro-, Phenyl- oder Phenoxyrest bedeutet, wobei der Phenyl- oder der Phenoxyrest durch Fluor, Chlor oder Brom oder Alkyl mit 1 bis 4 C-Atomen substituiert sein kann,
m gleich 1 oder 2 ist,
n gleich 0, 1 oder 2 ist und
Y ein Fluor- oder Chloratom bedeutet.

2. 1-(Trihalogenmethyl-sulfenyl)-4-methylimidazol-5-carboxamid der allgemeinen Formel I gemäß Anspruch 1, dadurch gekennzeichnet, daß R Wasserstoff, einen Methyl-, Ethyl-, n-Propyl- und Isopropylrest, und X ein Fluor-, Chlor- oder Bromatom oder einen Methoxy-, Ethoxy-, Phenoxy-, Methyl-, Ethyl-, tert.-Butyl-, Allyl-, Cyan-, Nitro-, Trifluormethyl- oder Phenylrest bedeutet.

3. Verfahren zur Herstellung von Verbindungen gemäß Anspruch 1, dadurch gekennzeichnet, daß man ein 4-Methyl-imidazol-5-carboxamid der Formel

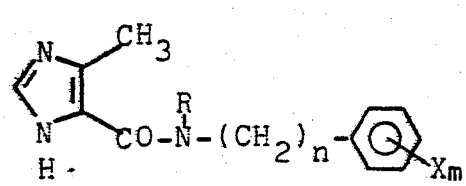 (II)

worin
R, X, m und n die in Anspruch 1 angegebene Bedeutung haben, mit einem Trihalogenmethyl-sulfenylchlorid der Formel

$$YCCl_2\text{-}S\text{-}Cl$$

worin
Y die in Anspruch 1 angegebene Bedeutung hat, vorzugsweise in Gegenwart eines Lösungsmittels und/oder Zusatz einer anorganischen oder organischen Base bei einer ausreichenden Temperatur unter 100°C umsetzt.

4. Verfahren zur Herstellung von Verbindungen gemäß Anspruch 1, wobei Y ein Fluoratom bedeutet, dadurch gekennzeichnet, daß man ein 1-(Trichlormethyl-sulfenyl)-4-methyl-imidazol-5-carboxamid der Formel

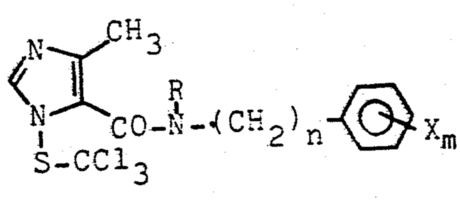 (Ia)

worin
R, X, m und n die in Anspruch 1 angegebene Bedeutung haben, mit wasserfreier Fluorwasserstoffsäure bei einer Temperatur unter +80°C gegebenenfalls unter Druck umsetzt.

5. Fungizid, enthaltend eine Verbindung gemäß Anspruch 1.

6. Fungizid, enthaltend einen inerten Zusatzstoff und eine Verbindung der Formel I gemäß Anspruch 1.

7. Verwendung einer Verbindung gemäß Anspruch 1 zur Bekämpfung von Pilzen.

8. Verfahren zur Bekämpfung von Pilzen, dadurch gekennzeichnet, daß man eine Verbindung der Formel I, gemäß Anspruch 1 auf diese bzw. auf durch Pilzbefall bedrohte Flächen, Pflanzen oder Saatgüter einwirken läßt.

9. Verfahren zur Herstellung eines fungiziden Mittels, dadurch gekennzeichnet, daß man eine Verbindung der Formel I, gemäß Anspruch 1 mit einem festen oder flüssigen Trägerstoff und gegebenenfalls mit einem oberflächenaktiven Mittel mischt.

Patentansprüche

1. Fungizid, enthaltend ein 1-(Trihalogenmethyl-sulfenyl)-4-methylimidazol-5-carboxamid der allgemeinen Formel

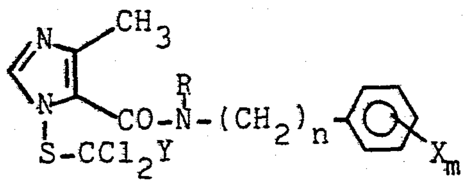

(I)

in der
R ein Wasserstoffatom oder einen Methyl-, Ethyl- oder Propylrest bedeutet,
X ein Wasserstoff- oder Halogenatom, einen gegebenenfalls durch Fluor, Chlor oder Brom substituierten Alkyl- oder Alkoxyrest mit 1 bis 5 C-Atomen, einen Alkenylrest mit 2 bis 4 C-Atomen, oder einen Cyan-, Nitro-, Phenyl- oder Phenoxyrest bedeutet, wobei der Phenyl- oder der Phenoxyrest durch Fluor, Chlor oder Brom oder Alkyl mit 1 bis 4 C-Atomen substituiert sein kann,
m gleich 1 oder 2 ist,
n gleich 0, 1 oder 2 ist und
Y ein Fluor- oder Chloratom bedeutet.

2. Fungizid gemäß Anspruch 1, dadurch gekennzeichnet, daß R Wasserstoff, einen Methyl-, Ethyl-, n-Propyl- und Isopropylrest, und X ein Fluor-, Chlor- oder Bromatom oder einen Methoxy-, Ethoxy-, Phenoxy-, Methyl-, Ethyl-, tert.-Butyl-, Allyl-, Cyan-, Nitro-, Trifluormethyl- oder Phenylrest bedeutet.

3. Verfahren zur Herstellung von Verbindungen gemäß Anspruch 1, dadurch gekennzeichnet, daß man ein 4-Methyl-imidazol-5-carboxamid der Formel

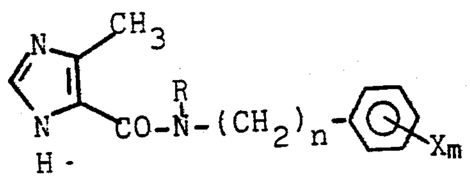

(II)

worin
R, X, m und n die in Anspruch 1 angegebene Bedeutung haben, mit einem Trihalogenmethyl-sulfenylchlorid der Formel

$$YCCl_2-S-Cl$$

worin
Y die in Anspruch 1 angegebene Bedeutung hat, vorzugsweise in Gegenwart eines Lösungsmittels und/oder Zusatz einer anorganischen oder organischen Base bei einer ausreichenden Temperatur unter 100°C umsetzt.

4. Verfahren zur Herstellung von Verbindungen gemäß Anspruch 1, wobei Y ein Fluoratom bedeutet, dadurch gekennzeichnet, daß man ein 1-(Trichlormethyl-sulfenyl)-4-methyl-imidazol-5-carboxamid der Formel

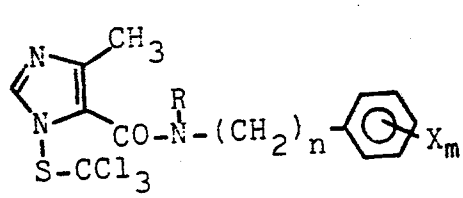

(Ia)

worin
R, X, m und n die in Anspruch 1 angegebene Bedeutung haben, mit wasserfreier Fluorwasserstoffsäure bei einer Temperatur unter +80°C gegebenenfalls unter Druck umsetzt.

5. Fungizid, enthaltend einen inerten Zusatzstoff und eine Verbindung der Formel I gemäß Anspruch 1.

6. Verwendung einer Verbindung gemäß Anspruch 1 zur Bekämpfung von Pilzen.

7. Verfahren zur Bekämpfung von Pilzen, dadurch gekennzeichnet, daß man eine Verbindung der Formel I, gemäß Anspruch 1 auf diese bzw. auf durch Pilzbefall bedrohte Flächen, Pflanzen oder Saatgüter einwirken läßt.

8. Verfahren zur Herstellung eines fungiziden Mittels, dadurch gekennzeichnet, daß man eine Verbindung der Formel I, gemäß Anspruch 1 mit einem festen oder flüssigen Trägerstoff und gegebenenfalls mit einem oberflächenaktiven Mittel mischt.